Europäisches Patentamt

European Patent Office

Office européen des brevets

(11) Veröffentlichungsnummer: **0 127 087**
**B1**

## (12) EUROPÄISCHE PATENTSCHRIFT

(45) Veröffentlichungstag der Patentschrift:
20.11.86

(51) Int. Cl.⁴: **C 07 C 7/17**

(21) Anmeldenummer: 84105654.2

(22) Anmeldetag: 18.05.84

(54) Verfahren zur Trennung eines im wesentlichen n-Butene und Butane enthaltenden C4-Kohlenwasserstoffgemisches.

(30) Priorität: 25.05.83 DE 3318858

(43) Veröffentlichungstag der Anmeldung:
05.12.84 Patentblatt 84/49

(45) Bekanntmachung des Hinweises auf die Patenterteilung:
20.11.86 Patentblatt 86/47

(84) Benannte Vertragsstaaten:
AT BE DE FR GB IT NL

(56) Entgegenhaltungen:
DE - A - 1 443 050
US - A - 3 068 305

(73) Patentinhaber: BASF Aktiengesellschaft,
Carl-Bosch-Strasse 38, D-6700 Ludwigshafen (DE)

(72) Erfinder: Lindner, Alfred, Dr., Ringstrasse 30,
D-6712 Bobenheim-Roxheim (DE)
Erfinder: Broellos, Klaus, Dr., Floriansring 7,
D-6104 Seeheim-Jungenheim (DE)
Erfinder: Sandrock, Gerhard, Dr.,
Albrecht-Duerer-Ring 36c, D-6710 Frankenthal (DE)
Erfinder: Volkamer, Klaus, Dr., Heidelberger Ring 21,
D-6710 Frankenthal (DE)
Erfinder: Hefner, Werner, Dr., Danziger Strasse 1c,
D-6840 Lampertheim (DE)

## Beschreibung

Die vorliegende Erfindung betrifft ein Verfahren zur Trennung eines im wesentlichen n-Butene und Butane enthaltenden $C_4$-Kohlenwasserstoffgemisches durch Umsetzung des Gemisches mit einer Carbonsäure, Abtrennung der Butane von dem gebildeten Ester, Zerlegung des Esters bei erhöhten Temperaturen in n-Butene und Carbonsäure und Abtrennung der n-Butene.

Es ist bekannt, n-Butene von Butanen durch extraktive Destillation unter Verwendung eines selektiven Lösungsmittels wie Furfurol zu trennen, wobei ein die Butane enthaltendes Destillat und ein die n-Butene enthaltender Extrakt erhalten werden. Die n-Butene werden in einem Ausgaser aus dem Extrakt abgetrennt, und das entgaste Lösungsmittel wird in die extraktive Destillation zurückgeführt. Dieses Verfahren ist apparativ sehr aufwendig, da wegen der eng beieinander liegender Siedepunkte der Butane und n-Butene und der geringen Löslichkeitsunterschiede im selektiven Lösungsmittel sehr hohe Kolonnen mit beispielsweise 200 Böden für die extraktive Destillation erforderlich sind.

In der deutschen Offenlegungsschrift 1 443 050 wird weiter ein Verfahren zur Abtrennung von Isobuten aus einem Isobuten, n-Butene und Butane enthaltenden $C_4$-Kohlenwasserstoffgemisch beschrieben, bei dem das im $C_4$-Kohlenwasserstoffgemisch enthaltene Isobuten in einer Veresterungsstufe mit einer Carbonsäure in Gegenwart eines Katalysators unter Bildung des Carbonsäure-tert.-butylesters umgesetzt wird und der erhaltene Carbonsäure-tert.-butylester anschliessend in einer zweiten Stufe in Carbonsäure und Isobuten zerlegt wird. Eine Umsetzung der ebenfalls in dem $C_4$-Kohlenwasserstoffgemisch in erheblichem Anteil enthaltenen n-Butene wird nicht erhalten.

Es wurde nun ein vorteilhaftes Verfahren gefunden zur Trennung eines im wesentlichen n-Butene und Butane enthaltenden $C_4$-Kohlenwasserstoffgemisches, welches dadurch gekennzeichnet ist, dass man

- das Gemisch mit einer Carbonsäure bei Temperaturen von 60 bis 120°C in Gegenwart eines sauren Katalysators unter Bildung des Carbonsäurebutylesters umsetzt,

- das aus der Reaktionszone für die Veresterung erhaltene Reaktionsgemisch anschliessend destilliert, wobei als Kopfprodukt eine die Butane enthaltende Fraktion und als Bodenprodukt eine den gebildeten Carbonsäurebutylester enthaltende Fraktion erhalten werden,

- danach den Carbonsäurebutylester bei erhöhten Temperaturen in Carbonsäure und n-Butene zerlegt und

- das Gemisch aus n-Butenen und Carbonsäure destilliert, wobei als Kopfprodukt die n-Butene und als Bodenprodukt die Carbonsäure erhalten werden.

Nach dem neuen Verfahren lassen sich n-Butene und Butane enthaltende $C_4$-Kohlenwasserstoffgemische mit geringem destillativem Aufwand durch einfache konventionelle Destillation in Destillationskolonnen mit relativ geringer Bodenzahl in eine n-Butene-Fraktion und eine Butane-Fraktion auftrennen.

Für das erfindungsgemässe Verfahren werden im wesentlichen n-Butene (d.h. 1-Buten und/oder 2-Buten-trans und/oder 2-Buten-cis) und Butane (d.h. Isobutan und/oder n-Butan) enthaltende $C_4$-Kohlenwasserstoffgemische als Ausgangsstoffe eingesetzt. Zweckmässig beträgt der Gehalt der Ausgangs-$C_4$-Kohlenwasserstoffgemische an den n-Butenen und Butanen mindestens 90 Gew.-%, vorzugsweise mindestens 94 Gew.-%, insbesondere mindestens 98 Gew.-%. Im allgemeinen sind die n-Butene in den Ausgangs-$C_4$-Kohlenwasserstoffen zu 1 bis 99 Gew.-%, vorzugsweise zu mindestens 10 Gew.-%, insbesondere zu mindestens 20 Gew.-%, enthalten. Solche Ausgangs-$C_4$-Kohlenwasserstoffgemische werden beispielsweise erhalten, indem aus einer $C_4$-Fraktion aus einer Ethylenanlage zunächst durch extraktive Destillation mit Hilfe eines selektiven Lösungsmittels Butadien extrahiert wird und anschliessend, z.B. unter Gewinnung des Methyl-tert.-butylethers, Isobutylen abgetrennt wird.

Das Ausgangs-$C_4$-Kohlenwasserstoffgemisch wird in der Veresterungsstufe mit einer Carbonsäure in Gegenwart eines sauren Katalysators unter Bildung des Carbonsäurebutylesters umgesetzt. Als Carbonsäuren kommen beispielsweise aliphatische Carbonsäuren mit zweckmässig 1 bis 8 Kohlenstoffatomen, vorzugsweise 1 bis 6 Kohlenstoffatomen, insbesondere 1 bis 4 Kohlenstoffatomen, in Betracht. Geeignete aliphatische Carbonsäuren sind beispielsweise Ameisensäure, Essigsäure, Propionsäure, n-Buttersäure, Isobuttersäure, n-Valeriansäure, Trimethylessigsäure, Capronsäure, n-Heptylsäure, Caprylsäure, Chloressigsäure. Mit besonderem Vorteil werden Essigsäure und insbesondere Propionsäure verwendet. Die Carbonsäuren werden z.B. als technische Produkte üblicher Reinheit, beispielsweise mit einer Reinheit von mindestens 95%, vorzugsweise mindestens 98% verwendet.

Als saure Katalysatoren für die Veresterung kommen beispielsweise Mineralsäuren wie Schwefelsäure, Phosphorsäure, organische Sulfonsäuren wie Benzolsulfonsäure, p-Toluolsulfonsäure, saure Aluminiumsalze oder saure Katalysatoren vom Friedel-Crafts-Typ wie Kupfer-(II)-chlorid, Eisen-(II)-chlorid sowie vorzugsweise Ionenaustauscher in der Wasserstofform in Betracht. Geeignete Ionenaustauscher sind beispielsweise sulfonierte Kohlen, sulfonierte Phenol-Formaldehyd-Harze, sulfonierte von Cumaron-Inden-Kondensationsprodukten abgeleitete Harze sowie insbesondere sulfonierte Polystyrol-Harze wie Kern-sulfonierte vernetzte Styrol-Divinylbenzol-Copolymerisate. Bei Verwendung eines flüssigen oder gelösten sauren Katalysators beträgt die Menge des flüssigen oder gelösten sauren Katalysators zweckmässig 0,001 bis 0,9 $\ell$, vorzugsweise 0,01 bis 0,7 $\ell$ pro $\ell$ Reaktorvolumen. Bei Verwendung von festen sauren Kondensationsmitteln beträgt die Menge des festen Kondensationsmittels zweckmässig 0,01 bis 1 $\ell$ Schüttvolumen pro $\ell$ Reaktorvolumen. Die festen sauren Kondensationsmittel können als solche oder auf einem Trägermaterial verwendet werden.

Geeignete Trägermaterialien sind z.B. Aluminiumoxid, Kieselsäure, Aktivkohle. Für die Veresterung können z.B. Rührkessel- oder Festbettreaktoren verwendet werden. Vorzugsweise werden Festbettreaktoren eingesetzt.

Für die Veresterung wird das Ausgangs-$C_4$-Kohlenwasserstoffgemisch mit der Carbonsäure in Gegenwart des sauren Katalysators bei Temperaturen von 60 bis 120°C umgesetzt. Bei Verwendung von Ionenaustauschern in der Wasserstofform als saure Katalysatoren werden zweckmässig Temperaturen von 60 bis 110°C angewendet.

Die erfindungsgemässe Veresterung kann bei Normaldruck erfolgen. Es ist jedoch zweckmässig, einen geringen Überdruck, z.B. Drücke von 1,01 bis 40 bar, insbesondere 4 bis 30 bar, anzuwenden. Das Ausgangs-$C_4$-Kohlenwasserstoffgemisch kann dabei je nach Druck und Temperatur für die Umsetzung flüssig oder gasförmig eingesetzt werden. Vorzugsweise werden flüssige Ausgangs-$C_4$-Kohlenwasserstoffgemische eingesetzt. Die Veresterung kann diskontinuierlich durchgeführt werden. Die Reaktionszeiten liegen bei diskontinuierlicher Arbeitsweise zweckmässig zwischen 1 Minute und 5 Stunden. Vorzugsweise wird die Veresterung jedoch kontinuierlich durchgeführt, wobei das Verhältnis aus Reaktorvolumen in ℓ und dem Durchsatz in ℓ/h zweckmässig 0,01 bis 5 Stunden, vorzugsweise 0,3 bis 1 Stunden beträgt.

Das Gewichtsverhältnis von Carbonsäure zu den im Ausgangs-$C_4$-Kohlenwasserstoffgemisch enthaltenen n-Butenen beträgt bei der Veresterung zweckmässig 100 : 1 bis 1 : 2, vorzugsweise 20 : 1 bis 1,1 : 1, insbesondere 4 : 1 bis 1 : 1. Der Umsatz der n-Butene beträgt zweckmässig 50 bis 90%, vorzugsweise 70 bis 80%.

Das nach der Veresterung erhaltene Reaktionsgemisch, welches in der Regel noch im Überschuss zur Veresterung zugesetzte Carbonsäure enthält, wird anschliessend destilliert, wobei als Kopfprodukt eine die Butane enthaltende Fraktion erhalten wird, welche die Butane und die nicht umgesetzten n-Butene enthält. Falls eine Butane enthaltende Fraktion mit einem möglichst geringen Gehalt an n-Butenen gefordert wird, kann es vorteilhaft sein, die erhaltene Butane enthaltende Fraktion einer weiteren Veresterung zu unterwerfen. Der Umsatz der n-Butene kann so auf 80 bis 95% gesteigert werden.

Als Bodenprodukt der Destillation des nach der Veresterung erhaltenen Reaktionsgemisches wird eine den gebildeten Carbonsäurebutylester enthaltende Fraktion erhalten, die gegebenenfalls noch im Überschuss zur Veresterung zugesetzte Carbonsäure enthält.

Der erhaltene Carbonsäurebutylester wird anschliessend in der zweiten Stufe des Verfahrens bei erhöhten Temperaturen in Carbonsäure und n-Butene zerlegt. Als Ausgangsprodukt für die Zerlegung kann Carbonsäurebutylester, der praktisch frei von Carbonsäure ist, verwendet werden, der beispielsweise durch Verwendung einer Menge an Carbonsäure bei der Veresterung, die höchstens der stöchiometrisch erforderlichen Carbonsäuremenge entspricht, oder durch Abtrennung, z.B. durch Abdestillieren, von überschüssig zugesetzter Carbonsäure aus dem nach der Destillation des Reaktionsgemischs der Veresterung erhaltenen Bodenprodukt erhalten worden ist. Vorzugsweise wird der nach der destillativen Abtrennung der Butane-Fraktion als Bodenprodukt erhaltene Carbonsäurebutylester ohne weitere Abtrennung von gegebenenfalls vorhandener überschüssiger Carbonsäure für die Zerlegung eingesetzt. Es ist jedoch auch möglich, nur einen Teil der überschüssigen Carbonsäure abzutrennen.

Für die Zerlegung wird der Carbonsäurebutylester zweckmässig verdampft und bei erhöhten Temperaturen in Carbonsäure und n-Butene zerlegt. Diese Zerlegung kann als thermische Spaltung, d.h. als Pyrolyse in Abwesenheit von Katalysatoren durchgeführt werden. Es kann jedoch auch vorteilhaft sein, die Zerlegung der Carbonsäureester in Gegenwart eines Katalysators durchzuführen.

Als Katalysatoren werden im allgemeinen saure Katalysatoren verwendet. Als saure Katalysatoren kommen beispielsweise Ionenaustauscher in der Wasserstofform, wie sulfonierte Kohlen, sulfonierte Phenol-Formaldehydharze, sulfonierte von Cumaron-Inden-Kondensationsprodukten abgeleitete Harze sowie sulfonierte Polystyrolharze wie Kernsulfonierte, vernetzte Styrol-Divinylbenzol-Copolymerisate in Betracht.

Weiter sind feste Phosphorsäurekatalysatoren, die Mono- oder vorzugsweise Polyphosphorsäure auf einem festen Trägermaterial enthalten, vorteilhaft geeignet. Geeignete Trägermaterialien für die Phosphorsäurekatalysatoren sind z.B. Aluminiumoxid, Kieselsäure, Aktivkohle, Kieselgur oder Bims. Vorzugsweise wird Kieselgel als Trägermaterial verwendet.

Weitere geeignete saure Katalysatoren sind saure Metallsulfate wie Natriumhydrogensulfat, Calciumhydrogensulfat, Aluminiumsulfate, Nickelsulfat, Kupfersulfat, Kobaltsulfat, Cadmiumsulfat, Strontiumsulfat. Diese sauren Metallsulfate können als solche verwendet werden. Vorzugsweise werden sie auf einem Trägermaterial angewendet. Geeignete Trägermaterialien sind z.B. Kieselgel, Aktivkohle, Aluminiumoxid oder Bims.

In einer weiteren Ausführungsform des erfindungsgemässen Verfahrens wird für die Zerlegung als saurer Katalysator ein Metallphosphat, insbesondere ein Metallhydrogenphosphat, verwendet. Diese Phosphate können Phosphorsäure auch im Überschuss, der über die stöchiometrische Zusammensetzung der sauren Metallphosphate hinausgeht, enthalten, z.B. in einem Überschuss bis zu 65%, vorzugsweise bis zu 20%, insbesondere bis zu 10%. Als derartige Metallphosphate können beispielsweise Magnesiumphosphate, Calciumphosphate, Strontiumphosphate, Bariumphosphate, Manganphosphate, Nickelphosphate, Kupferphosphate, Kobaltphosphate, Cadmiumphosphate, Eisen(II)-phosphate, Chromphosphate und insbesondere Aluminiumphosphate verwendet werden. Der Metallphosphat-Katalysator kann als solcher oder auf einem Trägermaterial verwendet werden. Geeignete Trägermaterialien sind beispielsweise Aluminiumoxid, Kieselsäure, Aktivkohle, Zinkoxid.

Die Menge des Katalysators beträgt zweckmässig 0,01 bis 2 kg, vorzugsweise etwa 0,01 bis 1 kg je 1 kg/h Durchsatz des Carbonsäurebutylesters durch den Reaktor. Vorzugsweise werden für die Zerlegung des Carbonsäurebutylesters in Gegenwart eines Katalysators Festbettreaktoren verwendet.

Die Zerlegungstemperatur des Carbonsäurebutylesters variiert, je nachdem, ob die Zerlegung an einem Katalysator oder ohne Katalysator durchgeführt wird, und in Abhängigkeit von der Reaktionszeit und bei Verwendung eines Katalysators von der Art des Katalysators. Bei der thermischen Zerlegung ohne Verwendung eines Katalysators werden zweckmässig Temperaturen von 160 bis 500 °C, vorzugsweise 170 bis 480 °C, insbesondere 200 bis 460 °C, angewendet. Bei Verwendung eines Katalysators liegt die Zerlegungstemperatur zweckmässig bei Temperaturen von 150 bis 480 °C, vorzugsweise 160 bis 400 °C, insbesondere 180 bis 350 °C.

Die Reaktionszeit des verdampften Carbonsäurebutylesters beträgt zweckmässig 0,1 bis 20 Sekunden, vorzugsweise 1 bis 10 Sekunden. Die Zerlegung des Carbonsäurebutylesters kann bei Normaldruck erfolgen. Es ist jedoch auch möglich, Überdruck anzuwenden, z.B. Drücke bis zu 30 bar, vorzugsweise bis zu 20 bar, insbesondere Drücke von 1 bis 10 bar. Die Zerlegung kann jedoch auch bei vermindertem Druck durchgeführt werden.

Die Zerlegung des Carbonsäurebutylesters kann diskontinuierlich erfolgen. Vorzugsweise wird sie jedoch kontinuierlich durchgeführt.

Das bei der Zerlegung erhaltene Reaktionsgemisch, welches als Reaktionsprodukte n-Butene und Carbonsäure enthält, wird anschliessend destilliert, wobei als Kopfprodukt die n-Butene und als Bodenprodukt die Carbonsäure erhalten werden. Die aus der Auftrennung erhaltene Carbonsäure wird zweckmässig in die Reaktionszone für die Veresterung zurückgeführt.

Aus dem erhaltenen Gemisch der n-Butene können durch destillative Auftrennung 1-Buten und 2-Buten gewonnen werden. 1-Buten ist ein wichtiger Ausgangsstoff, z.B. für die Herstellung von Polymeren wie Polybuten-1 sowie zur Herstellung von Butenoxid.

Das folgende Beispiel veranschaulicht die Erfindung.

*Beispiel*

Das für die Veresterung eingesetzte Ausgangs-$C_4$-Kohlenwasserstoffgemisch wurde erhalten, indem aus einer $C_4$-Fraktion aus einer Ethylenanlage zunächst durch extraktive Destillation Butadien extrahiert wurde und anschliessend unter Gewinnung des Methyl-tert.-butylethers Isobuten abgetrennt wurde. Die Zusammensetzung des $C_4$-Kohlenwasserstoffgemisches nach der Abtrennung von Butadien und Isobuten war wie folgt:

| 1-Buten | 46 Gew.-% |
|---|---|
| 2-Buten-trans | 16 Gew.-% |
| 2-Buten-cis | 11 Gew.-% |
| Isobuten | 3 Gew.-% |
| n-Butan | 20 Gew.-% |
| Isobutan | 4 Gew.-% |

Eine Mischung von 120 g je Stunde dieses $C_4$-Kohlenwasserstoffgemisches mit 130 g je Stunde Propionsäure wurde in einen Reaktor aus rostfreiem Stahl eingeleitet, in dem sich 120 ml eines sulfonierten Polystyroldivinylbenzol-Harzes in der Wasserstofform (Lewatit SPC 118, Kornfraktion 0,1 bis 1 mm) befanden. In dem Reaktor wurden eine Reaktionstemperatur von 100 °C und ein Druck von 20 bar aufrechterhalten. Das erhaltene Reaktionsgemisch wurde einer Destillationskolonne zugeführt, wobei am Kopf der Kolonne ein Butane-Gemisch erhalten wurde. Am Sumpf der Destillationskolonne wurden 200 g je Stunde Propionsäurebutylester, der noch 18,5 Gew.-%, bezogen auf das Sumpfprodukt, im Überschuss zugesetzte Propionsäure enthielt, abgezogen und in einen Verdampfer eingeleitet. Der verdampfte, auf 190 °C erhitzte Propionsäurebutylester wurde zur Zerlegung in einen rohrförmigen Spaltreaktor, welcher als Katalysator einen Phosphorsäure/Kieselgel-Trägerkatalysator enthielt, eingeleitet und bei 260 °C in n-Butene und Propionsäure aufgespalten. Das Reaktionsprodukt der Esterspaltung wurde in eine zweite Destillationskolonne eingeleitet, in der am Kopf 60 g je Stunde n-Buten-Gemisch der folgenden Zusammensetzung erhalten wurde:

| 1-Buten | 17,1 Gew.-% |
|---|---|
| 2-Buten-trans | 37,3 Gew.-% |
| 2-Buten-cis | 45,5 Gew.-% |
| Butan | 0,1 Gew.-% |

Die Ausbeute an n-Butenen, bezogen auf die im eingesetzten $C_4$-Kohlenwasserstoffgemisch enthaltenen n-Butene betrug 66 %. Am Sumpf der zweiten Destillationskolonne wurden Propionsäure erhalten, die noch Ester enthielt.

Arbeitete man wie oben beschrieben, führte jedoch die Veresterung zweistufig aus und führte die anschliessend in der Zerlegungsstufe zurückgewonnene, noch Propionsäurebutylester enthaltende Propionsäure in die Veresterung zurück, so liess sich die Ausbeute an n-Butenen auf über 90 % steigern.

**Patentansprüche**

1. Verfahren zur Trennung eines im wesentlichen n-Butene und Butane enthaltenden $C_4$-Kohlenwasserstoffgemisches, dadurch gekennzeichnet, dass man
   - das Gemisch mit einer Carbonsäure bei Temperaturen von 60 bis 120 °C in Gegenwart eines sauren Katalysators unter Bildung des Carbonsäurebutylesters umsetzt,
   - das aus der Reaktionszone für die Veresterung erhaltene Reaktionsgemisch anschliessend destilliert, wobei als Kopfprodukt eine die Butane enthaltende Fraktion und als Bodenprodukt eine den gebildeten Carbonsäurebutylester enthaltende Fraktion erhalten werden,
   - danach den Carbonsäurebutylester bei erhöhten Temperaturen in Carbonsäure und n-Butene zerlegt und
   - das Gemisch aus n-Butenen und Carbonsäure destilliert, wobei als Kopfprodukt die n-Butene und als Bodenprodukt die Carbonsäure erhalten werden.

2. Verfahren nach Anspruch 1, dadurch gekennzeichnet, dass man die nach der Zerlegung des Carbonsäurebutylesters zurückgewonnene Carbonsäure in die Reaktionszone für die Veresterung zurückführt.

3. Verfahren nach Anspruch 1 und 2, dadurch gekennzeichnet, dass man als Säure Propionsäure verwendet.

4. Verfahren nach Anspruch 1 bis 3, dadurch gekennzeichnet, dass man als sauren Katalysator für die Veresterung Ionenaustauscher in der Wasserstofform verwendet.

5. Verfahren nach Anspruch 1 bis 4, dadurch gekennzeichnet, dass man die Zerlegung des Carbonsäurebutylesters in Gegenwart eines Katalysators durchführt.

6. Verfahren nach Anspruch 5, dadurch gekennzeichnet, dass man als Katalysator für die Zerlegung einen Phosphorsäure/Kieselgel-Trägerkatalysator verwendet.

7. Verfahren nach Anspruch 1 bis 6, dadurch gekennzeichnet, dass die Zerlegung des Carbonsäurebutylesters bei Temperaturen von 200 bis 500°C durchgeführt wird.

## Claims

1. A process for separating a $C_4$-hydrocarbon mixture which essentially contains n-butenes and butanes, wherein

- the mixture is reacted with a carboxylic acid at a temperature of from 60 to 120°C in the presence of an acidic catalyst to form a butyl carboxylate,

- the reaction mixture obtained from the esterification zone is distilled to give, as the overhead product, a fraction containing the butanes, and, as the bottoms product, a fraction containing the resulting butyl carboxylate,

- the butyl carboxylate is decomposed at elevated temperatures to give the carboxylic acid and n-butenes, and

- the mixture of n-butenes and carboxylic acid is then distilled, the n-butenes being obtained as the overhead product, and the carboxylic acid as the bottoms product.

2. A process as claimed in claim 1, wherein the carboxylic acid recovered after decomposition of the butyl carboxylate is recycled to the esterification zone.

3. A process as claimed in claims 1 and 2, wherein the acid used is propionic acid.

4. A process as claimed in claims 1 to 3, wherein the acidic catalyst used for the esterification is an ion exchanger in the hydrogen form.

5. A process as claimed in claims 1 to 4, wherein the decomposition of the butyl carboxylate is carried out in the presence of a catalyst.

6. A process as claimed in claim 5, wherein the catalyst used for the decomposition is a phosphoric acid/silica gel supported catalyst.

7. A process as claimed in claims 1 to 6, wherein the decomposition of the butyl carboxylate is carried out at from 200 to 500°C.

## Revendications

1. Procédé pour séparer un mélange d'hydrocarbures en $C_4$, constitué essentiellement de n-butènes et de butanes, caractérisé en ce que

- on fait réagir le mélange à des températures comprises entre 60 et 120°C, en présence d'un catalyseur acide, avec un acide carboxylique, obtenant le carboxylate de butyle,

- on soumet ensuite le mélange réactionnel, obtenu dans la zone d'estérification, à une distillation qui donne comme produit de tête une fraction renfermant les butanes et comme résidu une fraction contenant le carboxylate de butyle formé,

- on décompose ensuite le carboxylate de butyle à des températures accrues en acide carboxylique et n-butènes et

- on soumet le mélange de n-butènes et d'acide carboxylique à une distillation qui donne comme produit de tête les n-butènes et comme résidu l'acide carboxylique.

2. Procédé suivant la revendication 1, caractérisé en ce que l'acide carboxylique reformé par décomposition du carboxylate de butyle est recyclé dans la zone d'estérification.

3. Procédé suivant la revendication 1 et la revendication 2, caractérisé en ce que l'acide utilisé est l'acide propionique.

4. Procédé suivant les revendications 1 à 3, caractérisé en ce que le catalyseur pour la réaction d'estérification est un échangeur d'ions dans sa forme $H^+$.

5. Procédé suivant les revendications 1 à 4, caractérisé en ce que la décomposition du carboxylate de butyle est également réalisée en présence d'un catalyseur.

6. Procédé suivant la revendication 5, caractérisé en ce que le catalyseur pour la décompostion est un catalyseur à l'acide phosphorique sur support de gel de silice.

7. Procédé suivant les revendications 1 à 6, caractérisé en ce que la décomposition du carboxylate de butyle est effectuée à des températures de 200 à 500°C.